(19) **Europäisches Patentamt**
**European Patent Office**
**Office européen des brevets**

(11) **EP 4 640 312 A1**

(12) **EUROPEAN PATENT APPLICATION**
published in accordance with Art. 153(4) EPC

(43) Date of publication:
**29.10.2025 Bulletin 2025/44**

(21) Application number: **23906861.2**

(22) Date of filing: **13.12.2023**

(51) International Patent Classification (IPC):
**B01J 35/51** *(2024.01)* **B01J 23/887** *(2006.01)*
**B01J 23/888** *(2006.01)* **C07B 61/00** *(2006.01)*
**C07C 45/35** *(2006.01)* **C07C 47/22** *(2006.01)*
**C07C 51/25** *(2006.01)* **C07C 57/05** *(2006.01)*

(52) Cooperative Patent Classification (CPC):
**B01J 23/887; B01J 23/888; B01J 35/51;**
**C07B 61/00; C07C 45/35; C07C 47/22;**
**C07C 51/25; C07C 57/04**

(86) International application number:
**PCT/JP2023/044644**

(87) International publication number:
**WO 2024/135496 (27.06.2024 Gazette 2024/26)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **20.12.2022 JP 2022202842**

(71) Applicant: **Nippon Kayaku Kabushiki Kaisha**
**Tokyo 100-0005 (JP)**

(72) Inventors:
• **YASUDA Shogo**
**Sanyoonoda-shi, Yamaguchi 757-8686 (JP)**
• **KAGAWA Tsukasa**
**Sanyoonoda-shi, Yamaguchi 757-8686 (JP)**
• **OKUMURA Shigeki**
**Sanyoonoda-shi, Yamaguchi 757-8686 (JP)**

(74) Representative: **Gille Hrabal**
**Partnerschaftsgesellschaft mbB**
**Patentanwälte**
**Brucknerstraße 20**
**40593 Düsseldorf (DE)**

(54) **CATALYST AND METHOD FOR PRODUCING COMPOUND USING SAME**

(57) The present invention relates to a catalyst having a value of a sphericity parameter SPHT3 obtained by particle shape measurement by a dynamic image analysis method of 0.9820 or less. Here, the sphericity parameter SPHT3 is a value obtained by the following equation (I). $SPHT3 = 4\pi A/P^2$ ···(I) In the equation (I), A is the area of a particle photographed by the dynamic image analysis method, and P is the perimeter of the particle.

**FIG. 1**

EP 4 640 312 A1

**Description**

TECHNICAL FIELD

The invention of the present application relates to a novel catalyst having high activity and achieving obtaining a target product in a high yield and particularly to a catalyst capable of stably oxidatively producing, in a high yield, an unsaturated aldehyde, an unsaturated carboxylic acid, or a conjugated diene.

BACKGROUND ART

A method for producing a corresponding unsaturated aldehyde or unsaturated carboxylic acid using propylene, isobutylene, t-butyl alcohol, or the like as a raw material, or a catalytic gas phase oxidation method for producing 1,3-butadiene from butenes is widely practiced industrially. In particular, regarding the method for producing a corresponding unsaturated aldehyde or unsaturated carboxylic acid using propylene, isobutylene, t-butyl alcohol, or the like as a raw material, many reports have been made as means for improving the yield.

In particular, acrylic acid has been increasingly important as a raw material for water absorbent resins, adhesives, and the like. Thus, in recent years, improved performance of a catalyst for producing acrylic acid by subjecting acrolein as a raw material to a catalytic gas-phase oxidation reaction has been demanded. Accordingly, various companies have made various improvements to catalysts that can be used to produce acrylic acid in a high yield and in a stable manner over a long period of time, and for example, the following proposals have been made.

PTLs 1 to 3 disclose improvement in the catalyst composition or the like focusing on X-ray diffraction peaks of the catalytically active component. These catalysts have been proposed as a catalyst that achieves high activity and a high yield. Further, in PTLs 4 and 5, an improvement guideline for the purpose of improving the mechanical strength is shown, and the catalyst performance is improved through prevention of powdering during filling. In PTL 6, by adjusting the standard deviation of the particle diameter of the catalyst within a specific range, the long-term stability of the catalytic reaction is improved. PTL 7 proposes to produce a catalyst having both high catalyst performance and mechanical strength by controlling the relative centrifugal acceleration during molding using a tumbling granulator.

However, particularly in production of an unsaturated carboxylic acid, improvement of the catalytic activity is an important issue. This does not merely contribute to improvement in the yield. For example, when the temperature of the reaction bath at the initial stage of the reaction decreases in a plant using a catalyst, the energy cost for heating decreases. Moreover, because the adverse thermal effect on the catalyst is reduced, the deterioration is small from a long-term viewpoint, and stable operation and a high yield can be achieved for a long time.

There is no technique for achieving sufficient catalytic activity at the moment, also including PTLs 1-6.

CITATION LIST

PATENT LITERATURE

PTL 1: JPH08-299797A
PTL 2: JP2003-251184A
PTL 3: JP2015-120133A
PTL 4: JP2001-79408A
PTL 5: WO2012/073584
PTL 6: JP2009-214105A
PTL 7: JP2015-96497A

SUMMARY OF INVENTION

TECHNICAL PROBLEM

In view of the above circumstances, an object of the invention of the present application is to improve the catalytic activity in a method for producing a corresponding unsaturated aldehyde or unsaturated carboxylic acid using propylene, isobutylene, t-butyl alcohol, or the like as a raw material or a catalytic gas-phase oxidation method for producing 1,3-butadiene from a butene.

SOLUTION TO PROBLEM

As a result of intensive studies on the current situation and the object described above, the inventors of the present application have found that a specific catalyst shape parameter contributes to improvement in activity and made the invention of the present application.

That is, the present invention relates to the following 1) to 9).

1) A catalyst having a value of a sphericity parameter SPHT3 obtained by particle shape measurement by a dynamic image analysis method of 0.9820 or less.
Here, the sphericity parameter SPHT3 is a value obtained by the following equation (I).

$$SPHT3 = 4\pi A/P^2 \cdots (I)$$

In the equation (I) above, A is the area of a particle photographed by the dynamic image analysis method, and P is the perimeter of the particle.
2) The catalyst according to 1) above containing a catalytically active component represented by the following formula (1).

$$(Mo)_{12}(V)_a(W)_b(Cu)_c(Sb)_d(X)_e(Y)_f(Z)_g(O)_h \qquad (1)$$

(In the formula, Mo, V, W, Cu, Sb, and O represent molybdenum, vanadium, tungsten, copper, antimony, and oxygen, respectively; X represents at least one element selected from the group consisting of an alkali metal and thallium; Y represents at least one element selected from the group consisting of magnesium, calcium, strontium, barium, and zinc; Z represents at least one element selected from the group consisting of bismuth, tellurium, silver, selenium, silicon, aluminum, boron, niobium, cerium, tin, chromium, manganese, iron, cobalt, nickel, samarium, germanium, zirconium, titanium, tantalum, lead, indium, sulfur, palladium, gallium, lanthanum, and arsenic. a, b, c, d, e, f, g, and h represent the atomic ratios of the respective elements, where a satisfies $0 < a \leq 10.0$, b satisfies $0 \leq b \leq 10.0$, c satisfies $0 < c \leq 6.0$, d satisfies $0 \leq d \leq 10.0$, e satisfies $0 \leq e \leq 0.50$, f satisfies $0 \leq f \leq 1.0$, and g satisfies $0 \leq g < 6.0$, with respect to molybdenum atom being 12. h is the number of oxygen atoms necessary for satisfying the valence of each component.)
3) The catalyst according to 2) above in which, in the formula (1), $1.0 \leq a \leq 5.0$, $0.50 \leq b \leq 3.0$, $0.50 \leq c \leq 3.0$, and $0 < d \leq 2.0$ are satisfied.
4) The catalyst according to 1) above containing a catalytically active component represented by the following formula (2).

$$Mo_{12}Bi_{b1}Ni_{c1}Co_{d1}Fe_{e1}X_{f1}Y_{g1}Z_{h1}O_{i1} \cdots \qquad (2)$$

(In the formula, Mo, Bi, Ni, Co, and Fe represent molybdenum, bismuth, nickel, cobalt, and iron, respectively; X is at least one element selected from tungsten, antimony, tin, zinc, chromium, manganese, magnesium, calcium, silicon, aluminum, cerium, and titanium; Y is at least one element selected from sodium, potassium, cesium, rubidium, and thallium; Z belongs to Group 1 to Group 16 in the periodic table and means at least one element selected from elements other than above Mo, Bi, Ni, Co, Fe, X, and Y, b1, c1, d1, e1, f1, g1, h1, and i1 represent the numbers of atoms of molybdenum, bismuth, nickel, cobalt, iron, X, Y, Z, and oxygen, respectively, where $0 < b1 \leq 7$, $0 \leq c1 \leq 10$, $0 < d1 \leq 10$, $0 < c1+d1 \leq 20$, $0 < e1 \leq 5$, $0 \leq f1 \leq 2$, $0 \leq g1 \leq 3$, and $0 \leq h1 \leq 5$ are satisfied, and i1 is a value determined by the oxidation state of each element.)
5) The catalyst according to 4) above in which, in the formula (2), $0.50 \leq b1 \leq 1.7$, $1.0 \leq c1 \leq 3.3$, $5.0 \leq d1 \leq 6.8$, and $1.0 \leq e1 \leq 3.0$ are satisfied.
6) The catalyst according to any one of 1) to 5) above in which a catalytically active component is carried on an inert carrier.
7) The catalyst according to 6) above in which the inert carrier is silica, alumina, or a combination thereof.
8) The catalyst according to any one of 1) to 7) above, which is used for producing an unsaturated aldehyde, an unsaturated carboxylic acid, or a conjugated diene compound.
9) A method for producing an unsaturated aldehyde, an unsaturated carboxylic acid, or a conjugated diene compound using the catalyst according to any one of 1) to 8) above.

ADVANTAGEOUS EFFECTS OF INVENTION

According to the present invention, the catalytic activity can be maintained high in a method for producing a corresponding unsaturated aldehyde or unsaturated carboxylic acid using propylene, isobutylene, t-butyl alcohol, or the like as a raw material or a catalytic gas-phase oxidation method for producing 1,3-butadiene from a butene.

BRIEF DESCRIPTION OF DRAWINGS

FIG. 1 is a schematic view showing the method for adding the binder in Example 1.
FIG. 2 is a schematic view showing the method for adding the binder in Example 2.
FIG. 3 is a schematic view showing the method for adding the binder in Example 4.
FIG. 4 is a schematic view showing the method for adding the binder in Example 6.
FIG. 5 is a schematic view showing the method for adding the binder in Comparative Example 1.
FIG. 6 is a schematic view showing the method for adding the binder in Comparative Example 2.
FIG. 7 is a schematic view showing the method for adding the binder in Example 7.

DESCRIPTION OF EMBODIMENTS

[Particle Shape by Dynamic Image Analysis Method]

The catalyst of the present invention has a value of SPHT3 (sphericity parameter) obtained by particle shape measurement by a dynamic image analysis method of 0.9820 or less.

The dynamic image analysis method is a method of determining the particle size distribution and the shape distribution (aspect ratio, sphericity, and the like) by continuously photographing particles dispersed in a fluid (a solvent, air, or the like) and performing measurement, binarization, and analysis. Examples of a practically used device for performing the dynamic image analysis method include CAMSIZER X2 (manufactured by MicrotracBEL Corp) and the like.

In the present description, the sphericity and the sphericity parameter (SPHT3) are synonymous.

The sphericity SPHT3 is analyzed from continuously photographed particle images. The sphericity can be calculated by applying the perimeter P and the area A of the image taken for each particle to the following equation (I). In the case of the perfect sphere, SPHT3 is 1, and all particles having other shapes have a SPHT3 of less than 1 and larger than 0.

$$SPHT3 = 4\pi A/P^2 \cdots (I)$$

In the present invention, when the value of the SPHT3 of a catalyst is within a certain range, it means that the average value of the SPHT3 values measured for about 100 to 200 particles of the catalyst is within the range. The upper limit of the SPHT3 is further preferably 0.9810, 0.9805, 0.9800, 0.9770, or 0.9765 in this order, and particularly preferably 0.9760. The lower limit is about 0.8000, further preferably 0.8500, 0.9000, 0.9200, or 0.9310 in this order, and particularly preferably 0.9320. Therefore, the range of the SPHT3 is preferably 0.8000 or more and 0.9810 or less, more preferably 0.8500 or more and 0.9805 or less, more preferably 0.9000 or more and 0.9800 or less, more preferably 0.9200 or more and 0.9770 or less, more preferably 0.9310 or more and 0.9765 or less, and most preferably 0.9320 or more and 0.9760 or less.

The inventors of the present invention have found that a higher activity is exhibited as the shape of the catalyst deviates from the perfect spherical shape (that is, the SPHT3 is a value smaller than 1). It is believed that, when a catalytic reaction is conducted while a gas flows in a reaction tube filled with a catalyst, the contact time with the catalyst increases due to disturbance in the gas flow and retention in the catalyst layer as the shape of the catalyst deviates from the perfect spherical shape, and as a result, improvement of the conversion rate of the raw material is observed. As another factor, that the shape of the catalyst deviates from the perfect spherical shape means that the pore distribution of the spherical catalyst and/or the thickness of the active component are not uniform, and heat accumulation of local reaction heat is believed to occur.

[Symm3 (symmetry)]

The Symm3 in the present invention is a parameter indicating the geometric symmetry analyzed from images continuously taken by the dynamic image analysis method, and the calculation method thereof is as follows.

First, a straight line which passes through the center of gravity of an image taken for a particle and which intersects with the outer periphery of the particle is drawn in an optional direction. The distances from the center of gravity to the outer periphery of the particle are regarded as r1 and r2. The direction of the straight line is changed, and the smallest value of r1/r2 is defined as min (r1/r2). A value calculated by applying the value to the following equation (II) is defined as Symm3 (symmetry). The catalyst of the present invention further preferably has Symm3 of 0.9860 or less from the viewpoint of catalytic activity. The upper limit of the Symm3 is further preferably 0.9800, 0.9770, or 0.9760 in this order, and particularly preferably 0.9750. The lower limit is about 0.9600, further preferably 0.9650, and particularly preferably 0.9715. Therefore, the range of the Symm3 is more preferably 0.9600 or more and 0.9800 or less, more preferably 0.9600 or more and 0.9770 or less, more preferably 0.9650 or more and 0.9760 or less, and most preferably 0.9715 or more and 0.9750 or less.

$$Symm3 = \{1+min(r1/r2)\}/2 \cdots (II)$$

[Aspect Ratio]

The aspect ratio b/I3 in the present invention is a parameter indicating the shape of each particle continuously photographed by the dynamic image analysis method, and the calculation method thereof is as follows. A point on the outer periphery of a photographed particle is denoted by P, and a straight line is drawn from the point P in such a manner that the line passes through the inside of the particle and intersects with a point Q on the outer periphery different from the point P. The point Q is moved along the outer periphery, and the maximum value of the length of the line segment PQ is defined as a cord diameter $X_c$. The point P is moved along the outer periphery, and the minimum value of the chord diameter $X_c$ is defined as the minimum chord diameter $X_{cmin}$. Next, two parallel tangent lines sandwiching the particle are drawn, and the distance between the tangent lines is defined as Feret diameter $X_{Fe}$. The direction of the tangent lines is changed, and the maximum value of the Feret diameter $X_{Fe}$ is defined as the maximum Feret diameter $X_{FeMAX}$. The aspect ratio b/I3 is calculated by the following equation (III). The catalyst of the present invention further preferably has an aspect ratio b/I3 of 0.9540 or less in terms of activity. The upper limit of the aspect ratio is further preferably 0.9500, 0.9400, or 0.9300 in this order, and particularly preferably 0.9280. The lower limit is about 0.9200, and further preferably 0.9250. Therefore, the range of the aspect ratio is more preferably 0.9200 or more and 0.9540 or less, more preferably 0.9200 or more and 0.9500 or less, more preferably 0.9200 or more and 0.9400 or less, more preferably 0.9200 or more and 0.9300 or less, and most preferably 0.9250 or more and 0.9280 or less.

$$b/I3 = X_{cmin}/X_{FeMAX} \cdots (III)$$

[Catalyst Composition of Catalyst for Producing Unsaturated Carboxylic Acid]

When the catalyst of the present invention is used as a catalyst for producing an unsaturated carboxylic acid, the catalytically active component preferably has a composition represented by the following formula (1).

$$(Mo)_{12}(V)_a(W)_b(Cu)_c(Sb)_d(X)_e(Y)_f(Z)_g(O)_h \qquad (1)$$

(In the formula, Mo, V, W, Cu, Sb, and O represent molybdenum, vanadium, tungsten, copper, antimony, and oxygen, respectively, X represents at least one element selected from the group consisting of an alkali metal and thallium, Y represents at least one element selected from the group consisting of magnesium, calcium, strontium, barium, and zinc, and Z represents at least one element selected from the group consisting of bismuth, tellurium, silver, selenium, silicon, aluminum, boron, niobium, cerium, tin, chromium, manganese, iron, cobalt, nickel, samarium, germanium, zirconium, titanium, tantalum, lead, indium, sulfur, palladium, gallium, lanthanum, and arsenic. a, b, c, d, e, f, g, and h represent the atomic ratios of the respective elements, where a represents $0 < a \leq 10.0$, b represents $0 \leq b \leq 10.0$, c represents $0 < c \leq 6.0$, d represents $0 \leq d \leq 10.0$, e represents $0 \leq e \leq 0.50$, f represents $0 \leq f \leq 1.0$, and g represents $0 \leq g < 6.0$, with respect to molybdenum atom being 12. h is the number of oxygen atoms necessary for satisfying the valence of each component.)

In the above formula (1), preferred ranges of a to g are as follows.

The lower limit of a is 0.20, 0.50, 0.80, 1.0, 1.5, 2.0, 2.2, or 2.5 in order of desirability and is most desirably 2.8, and the upper limit of a is 9.0, 8.0, 7.0, 6.0, 5.0, 4.5, 4.0, or 3.5 in order of desirability and is most desirably 3.2. That is, the range of a is preferably $0.20 \leq a \leq 9.0$, more preferably $0.50 \leq a \leq 8.0$, more preferably $0.80 \leq a \leq 7.0$, more preferably $1.0 \leq a \leq 6.0$, more preferably $1.5 \leq a \leq 5.0$, more preferably $2.0 \leq a \leq 4.5$, more preferably $2.2 \leq a \leq 4.0$, more preferably $2.5 \leq a \leq 3.5$, and most preferably $2.8 \leq a \leq 3.2$.

The lower limit of b is 0.10, 0.20, 0.30, 0.40, 0.50, 0.60, 0.70, 0.80, or 0.90 in order of desirability and is most desirably 1.0, and the upper limit of b is 9.0, 8.0, 7.0, 6.0, 5.0, 4.0, 3.0, 2.5, 2.0, or 1.5 in order of desirability and is most desirably 1.4. That is, the range of b is preferably $0.10 \leq b \leq 9.0$, more preferably $0.10 \leq b \leq 8.0$, more preferably $0.20 \leq b \leq 7.0$, more preferably $0.30 \leq b \leq 6.0$, more preferably $0.40 \leq b \leq 5.0$, more preferably $0.50 \leq b \leq 4.0$, more preferably $0.60 \leq b \leq 3.0$, more preferably $0.70 \leq b \leq 2.5$, more preferably $0.80 \leq b \leq 2.0$, more preferably $0.90 \leq b \leq 1.5$, and most preferably $1.0 \leq b \leq 1.4$.

The lower limit of c is 0.10, 0.20, 0.30, 0.40, 0.50, 0.60, 0.70, 0.80, or 0.90 in order of desirability and is most desirably 1.0, and the upper limit of c is 5.0, 4.0, 3.0, 2.5, 2.0, or 1.5 in order of desirability and is most desirably 1.4. That is, the range of c is preferably $0.10 \leq c \leq 5.0$, more preferably $0.20 \leq c \leq 5.0$, more preferably $0.30 \leq c \leq 5.0$, more preferably $0.40 \leq c \leq 5.0$, more preferably $0.50 \leq c \leq 4.0$, more preferably $0.60 \leq c \leq 3.0$, more preferably $0.70 \leq c \leq 2.5$, more preferably $0.80 \leq c \leq 2.0$, more preferably $0.90 \leq c \leq 1.5$, and most preferably $1.0 \leq c \leq 1.4$.

The lower limit of d is 0.11, 0.15, 0.18, 0.20, 0.25, 0.30, or 0.35 in order of desirability and is most desirably 0.40, and the upper limit of d is 9.0, 8.0, 7.0, 6.0, 5.0, 4.0, 3.0, 2.5, 2.0, 1.5, or 1.0 in order of desirability and is most desirably 0.70. That is, the range of d is preferably $0.11 \leq d \leq 9.0$, more preferably $0.11 \leq d \leq 8.0$, more preferably $0.11 \leq d \leq 7.0$, more preferably $0.11 \leq d \leq 6.0$, more preferably $0.11 \leq d \leq 5.0$, more preferably $0.15 \leq d \leq 4.0$, more preferably $0.18 \leq d \leq 3.0$, more preferably $0.20 \leq d \leq 2.5$, more preferably $0.25 \leq d \leq 2.0$, more preferably $0.30 \leq d \leq 1.5$, more preferably $0.35 \leq d \leq 1.0$, and most preferably $0.40 \leq d \leq 0.70$.

The upper limit of e is 0.40, 0.30, 0.20, or 0.10 in order of desirability. That is, the range of e is $0 \leq e \leq 0.40$, $0 \leq e \leq 0.30$, or $0 \leq e \leq 0.20$ in order of preference, and the most preferable range is $0 \leq e \leq 0.10$.

The upper limit of f is 0.80, 0.50, 0.20, or 0.15 in order of desirability and is most desirably 0.10. That is, the range of f is $0 \leq f \leq 0.80$, $0 \leq f \leq 0.50$, $0 \leq f \leq 0.20$, or $0 \leq f \leq 0.15$ in order of preference, and the most preferable range is $0 \leq f \leq 0.10$.

The upper limit of g is 5.0, 4.0, 3.0, 2.0, or 1.0 in order of desirability. That is, the range of g is $0 \leq g \leq 5.0$, $0 \leq g \leq 4.0$, $0 \leq g \leq 3.0$, or $0 \leq g \leq 2.0$ in order of preference, and the most preferable range is $0 \leq g \leq 1.0$.

Here, a case in which e, f, and g are 0 is a particularly preferable aspect.

[Carrying]

The catalyst in which a preliminarily calcined product which is obtained by preliminary calcination after preparation of the catalytically active component or a preliminarily calcined powder after pulverization which is obtained by further subjecting the preliminarily calcined product to a pulverization step is carried on an inert carrier has a particularly excellent effect.

As the material of the inert carrier, known materials such as alumina, silica, titania, zirconia, niobia, silica-alumina, silicon carbide, carbide, and a mixture thereof can be used. The particle diameter, the water absorption, the mechanical strength, the crystallinity and the mixing ratio of each crystal phase, and the like of the inert carrier are not particularly limited, and appropriate ranges should be selected in consideration of the final catalyst performance, the moldability, the production efficiency, and the like. The mixing proportion of the carrier to the preliminarily calcined powder is basically calculated as a carrying ratio based on the charged mass of each raw material according to the following equation. When it is clear that the additives such as the molding aid and the strength improver used remain in the catalyst even after the main calcination, the additives are included in the total amount (denominator).

Carrying ratio (mass%) = (mass of preliminarily calcined powder for use in molding)/{(mass of preliminarily calcined powder for use in molding) + (mass of carrier for use in molding)} $\times$ 100

The upper limit of the carrying ratio is preferably 80 mass% and is 70, 60, 55, 50, 45, 40, 47, 45, 43, 40, 38, 37, 36, or 35 mass% in order of preference.

The lower limit thereof is preferably 10 mass% and is 15, 18, 20, 23, 25, 28, 30, 31, 32, or 33 mass% in order of preference. That is, the carrying ratio is preferably 10 mass% or more and 80 mass% or less, and the most preferred range is 33 mass% or more and 35 mass% or less.

The inert carrier is preferably silica and/or alumina, and particularly preferably a mixture of silica and alumina.

A binder is preferably used for carrying. Specific examples of the binder to be used include water, ethanol, methanol, propanol, a polyhydric alcohol, polyvinyl alcohol as a polymer-based binder, and a silica sol aqueous solution as an inorganic binder. Ethanol, methanol, propanol, and a polyhydric alcohol are preferred, a diol such as ethylene glycol and a triol such as glycerin are preferred, and an aqueous solution having a concentration of glycerin of 5 mass% or more is preferred. Using an appropriate amount of a glycerin aqueous solution allows for providing a high-performance catalyst having good molding properties and high mechanical strength. The amount of these binders to be used is typically 2 to 60 parts by mass with respect to 100 parts by mass of the preliminarily calcined powder, and 10 to 30 parts by mass is preferred in the case of using the glycerin aqueous solution. The binder and the preliminarily calcined powder may be alternately supplied to a molding machine or simultaneously supplied to the molding machine during carrying.

The carried catalyst obtained as described above can be directly supplied as a catalyst for the gas-phase catalytic oxidation reaction, and is preferably subjected to firing because the catalytic activity may be improved. The firing method and the firing conditions are not particularly limited, and known treatment methods and conditions can be applied. The optimum calcination conditions vary depending on the raw material for catalyst to be used, the catalyst composition, the preparation method, and the like. The calcination temperature is typically 100 to 450°C, preferably 270 to 420°C, particularly preferably 350 to 390°C, and the calcination time is one to 20 hours. The firing is typically carried out in an air atmosphere, and may be carried out in an atmosphere of an inert gas such as nitrogen, carbon dioxide, helium, or argon, or the firing may be carried out in an air atmosphere after firing in an inert gas atmosphere, if necessary.

Using the catalyst according to the present invention in a reaction that produces a corresponding unsaturated carboxylic acid using an unsaturated aldehyde such as acrolein and methacrolein as a raw material, particularly in a reaction that produces acrylic acid by subjecting acrolein to catalytic gas-phase oxidation with molecular oxygen or a molecular oxygen-containing gas, allows for achieving improvement in catalytic activity and the reduction in differential pressure, and is very effective as compared with the known method. Also in a process of a partial oxidation reaction accompanied by heat generation, an effect of improving the stability due to reduction of the hot spot temperature or the like can be expected. Further, the catalyst of the present invention is also effective in reducing by-products that adversely influence the environment and the quality of the final product, such as carbon monoxide (CO), carbon dioxide ($CO_2$), acetaldehyde, acetic acid, and formaldehyde.

In the method for producing an unsaturated carboxylic acid of the present invention, the method for circulating the raw material gas may be a normal single flow method or a recycling method, and the method can be carried out under generally used conditions and is not particularly limited. For example, a mixed gas containing 1 to 10 vol% and preferably 4 to 9 vol% of an unsaturated aldehyde as a starting raw material substance at normal temperature, 3 to 20 vol% and preferably 4 to 18 vol% of molecular oxygen, 0 to 60 vol% and preferably 4 to 50 vol% of water vapor, and 20 to 80 vol% and preferably 30 to 60 vol% of an inert gas such as carbon dioxide and nitrogen is introduced, at 240 to 450°C under normal pressure to pressure of 10 atm at a space velocity of 300 to 5000 $h^{-1}$, into the catalyst of the present invention packed in a reaction tube, and the reaction is carried out.

[Inorganic Fiber]

The catalyst of the present invention preferably contains inorganic fibers for the purpose of improving the mechanical strength or the like. The material of the inorganic fibers is not particularly limited, but for example, glass fibers, ceramic fibers, metal fibers, mineral fibers, carbon fibers, various whiskers, and the like can be used. Of these, glass fibers treated with a silane-based chemical product are particularly preferable.

The fiber length thereof is not particularly limited as long as it does not impair the effects of the present invention, but the average fiber length is preferably about 1 to 1000 $\mu$m, and more preferably about 10 to 500 $\mu$m.

Further, two or more types of the inorganic fibers can be used in combination, and two or more types having different materials or two types of a same material having different average fiber lengths may be used.

[Method for Producing Catalyst for Producing Unsaturated Carboxylic Acid, etc.]

Examples of specific steps for obtaining the catalyst for producing an unsaturated carboxylic acid of the present invention are shown below.

Step a) preparation

In general, regarding the raw material of each element constituting the catalyst, a high-performance catalyst can be obtained when ammonium molybdate is used as a molybdenum component raw material.

As raw materials for tungsten, vanadium, antimony, copper and other elements, typically, an oxide, or an ammonium salt, a carbonate salt, an organic acid salt, and a hydroxide, which can be converted into an oxide by high heat, or mixtures thereof can be used. For example, a vanadium component raw material, a molybdenum component raw material, and an antimony component raw material are mixed in a desired ratio with a separately prepared aqueous solution or slurry of a tungsten component raw material and a Z component raw material under conditions of 20 to 95°C, and the mixture is heated and stirred under conditions of 20 to 90°C for about one hour. Then, an aqueous solution in which a copper component raw material is dissolved and, if necessary, an X component raw material and a Y component raw material are added to obtain an aqueous solution or slurry containing the catalyst components. Hereinafter, the aqueous solution or the slurry obtained in this manner is collectively referred to as a prepared liquid (A). Here, the prepared liquid (A) is not always necessary to contain all the catalyst constituent elements, and some elements or some amounts thereof may be added in the subsequent steps. In addition, in the case of adding an amount of water for dissolving the component raw materials or an acid such as sulfuric acid, nitric acid, hydrochloric acid, tartaric acid, and acetic acid for dissolution when preparing the prepared liquid (A), unless the sufficient acid concentration in the aqueous solution to dissolve the raw materials is suitable, for example, in the range of 5 mass% to 99 mass%, the prepared liquid (A) sometimes becomes in the form of a clay-like lump, and this does not result in an excellent catalyst. Therefore, the form of the obtained prepared liquid (A) is preferably an aqueous solution or slurry as an excellent catalyst can be obtained.

Step b) drying

Next, the prepared liquid (A) obtained above is dried to form a dry powder. The drying method is not limited as long as it is a method capable of completely drying the prepared liquid (A), and examples thereof include drum drying, freeze drying, spray drying, and evaporation to dryness. Among these, in the present invention, spray drying is particularly preferred since the slurry can be dried into a powder or granules in a short period of time. The drying temperature in the spray drying varies depending on a concentration of the slurry, a liquid feeding rate, or the like, and the temperature at the outlet of a dryer is typically 70°C to 150°C. In addition, drying is preferably performed such that the average particle diameter of the dry powder obtained at this time is 20 to 700 $\mu$m. In this way, a dry powder (B) is obtained.

Step c) preliminary calcination

When the obtained dry powder (B) is calcined under air circulation at 200°C to 500°C, preferably at 300°C to 400°C, the moldability, the mechanical strength, and the catalyst performance of the catalyst tend to improve. The calcination time is preferably 1 hour to 12 hours. In this way, a preliminarily calcined product (C) is obtained.

Step d) pulverization

The obtained preliminarily calcined product (C) is sometimes obtained as a solid matter (D) in which the dry powder (B) is aggregated by preliminary calcination. The solid matter (D) is pulverized to obtain a preliminarily calcined powder (E) required in the next molding step. The pulverization method is not limited, but examples thereof include a roller mill, a jet mill, a hammer mill, a rotary mill, and a vibration mill. The average particle diameter (pre-calcined median diameter) of the preliminarily calcined powder (E) obtained at this time is preferably 50 $\mu$m or less, more preferably 40 $\mu$m or less, further preferably 30 $\mu$m or less, and particularly preferably 25 $\mu$m or less.

In the present description of this application, the preliminarily calcined powder (E) after pulverization is described as a catalyst precursor, but when there is no aggregation in the stage of the preliminarily calcined product (C) and the preliminarily calcined product (C) can be used without going through the pulverization step, the preliminarily calcined product (C) may be used as a catalyst precursor.

Step e) molding

The molding method is not particularly limited, and the preliminarily calcined powder (E) may be molded into a spherical shape using a molding machine, but a method of carrying the preliminarily calcined powder (E) (containing a molding aid and a strength improver if necessary) on a carrier such as inert ceramic is preferable. Here, as a carrying method, a tumbling granulation method, a method using a centrifugal fluid coating device, a wash coating method, and the like are widely known, and the method is not particularly limited as long as the preliminarily calcined powder (E) can be uniformly carried on the carrier. In consideration of the production efficiency of the catalyst and the performance of the prepared catalyst, more preferred is a method of rotating a flat or uneven disc at a high speed in an apparatus that includes the disc at a bottom portion of a fixed cylindrical container, to vigorously stir a carrier charged in the container by rotation and revolution movements of the carrier itself, and carrying the powder component on the carrier by adding, to the container, the preliminarily calcined powder (E), and if necessary a molding aid and/or a strength improver, and a pore-forming agent. A binder is preferably used for carrying. Specific examples of the binder to be used include water, ethanol, methanol, propanol, a polyhydric alcohol, polyvinyl alcohol that is a polymer-based binder, and a silica sol aqueous solution that is an inorganic binder. Ethanol, methanol, propanol, and a polyhydric alcohol are preferred, a diol such as ethylene glycol and a triol such as glycerin are more preferred. Using an appropriate amount of a glycerin aqueous solution allows for providing a high-performance catalyst having good molding properties and high mechanical strength. Specifically, a particularly high-performance catalyst can be obtained when an aqueous solution having a concentration of glycerin of 5 mass% or more is used. The amount of these binders to be used is typically 2 to 80 parts by mass with respect to 100 parts by mass of the preliminarily calcined powder (E). An inert carrier of about 2 to 8 mm is typically used, and the preliminarily calcined powder (E) is carried thereon. The carrying ratio is determined in consideration of the catalyst usage conditions, for example, reaction conditions such as the space velocity of the reaction raw materials and the concentrations of the raw materials and is typically 20 mass% to 80 mass%. Here, the carrying ratio is expressed as in the following equation (4) when a molding aid, a strength improver and the like are used in molding. In this way, a molded body (F) is obtained.

In addition, as found by the present inventors, it is difficult to maintain the mechanical strength in the present invention. Therefore, an inert inorganic fiber is preferably added as a strength improver, and particularly preferably, a glass fiber is used during carrying and molding. When a fiber is used as the strength improver, the amount of the fiber used is usually 1 to 30 parts by mass, preferably 2 to 10 parts by mass, and more preferably 3 to 5 parts by mass with respect to 100 parts by mass of the catalytically active component solid.

Here, as the material of the inert carrier, known materials such as alumina, silica, titania, zirconia, niobia, silica-alumina, silicon carbide, carbide, and a mixture thereof can be used. The particle diameter, the water absorption, the mechanical strength, the crystallinity and the mixing ratio of each crystal phase, and the like of the inert carrier are not particularly limited, and appropriate ranges should be selected in consideration of the final catalyst performance, the shaping property, the production efficiency, and the like.

[Equation (4)]

Carrying ratio (mass%)

= 100 × [mass of preliminarily calcined powder (E) used for molding/(mass of preliminarily calcined

powder (E) used for molding + mass of inert carrier used for molding)]

When it is clear that the additives such as the molding aid and the strength improver used remain in the catalyst even after the main calcination, the additives are included in the total amount (denominator).

Step f) main calcination

When the molded body (F) is calcined at a temperature of 100°C to 450°C for about 1 to 12 hours, the catalytic activity and the useful yield tend to improve. The calcination temperature is preferably 270°C or higher and 420°C or lower, and more preferably 350°C or higher and 400°C or lower. Air is convenient and preferred as a gas to be circulated. In addition, nitrogen, carbon dioxide, a nitrogen oxide-containing gas for creating a reducing atmosphere, an ammonia-containing gas, a hydrogen gas, and mixtures thereof can be also used as inert gases. In this way, a catalyst (G) is obtained.

[Method for Adjusting SPHT3]

The value of the SPHT3 above can be adjusted by changing the raw materials used in the step a), the spray drying condition in the step b), the calcination temperature and time in the step c), the pulverization method and the median diameter after pulverization in the step d), and the relative centrifugal acceleration, the carrying ratio, the type of the binder, the addition position of the binder, and the like in the step e). However, it is difficult to greatly change the value by changing a single condition, and the value can be achieved by optimizing two or more conditions. Some examples will be described below.

<Calcination Temperature and Time in Step c)>

The calcination temperature is preferably 200°C to 500°C as described above, but when the calcination temperature is increased, the SPHT3 tends to increase. Therefore, for adjusting the SPHT3, the calcination temperature is preferably lower than 380°C. Further, because the SPHT3 may also increase as the calcination time becomes longer, the calcination time is preferably shorter than five hours.

<Pulverization Method and Median Diameter in Step d)>

The SPHT3 can also be controlled by the method of the step d). When a vibration mill is used, the SPHT3 is easily adjusted. The SPHT3 can also be adjusted by adjusting the median diameter of the preliminarily calcined powder to a certain value or less.

<Relative Centrifugal Acceleration and Carrying Ratio in Step e)>

The value of the SPHT3 can also be adjusted by changing the relative centrifugal acceleration in the step e). The relative centrifugal acceleration may be about 2.0 G or more and 30 G or less, but when the relative centrifugal acceleration is increased, the SPHT3 tends to increase, and the relative centrifugal acceleration is most suitably about 2.0 G or more and 6.0 G or less. However, the relative centrifugal acceleration varies depending on the relation of the amounts of the carrier and the preliminarily calcined powder carried thereon (carrying ratio).

<Median Diameter in Step d) and Addition Position of Binder in Step e)>

The SPHT3 can also be controlled by adjusting the median diameter of the preliminarily calcined powder to a certain value or less by the method of the step d), but the SPHT3 can also be adjusted by changing the addition position of the binder in the step e). The binder is preferably added at a position away from the addition position of the granules, and the binder is preferably added at an even flow rate by providing a binder addition position in the middle of the doughnut-shaped catalyst band generated when the bottom plate of the tumbling granulator is rotated or providing a plurality of binder addition parts in the radius vector direction.

The adjustment methods of the SPHT3 also apply to the adjustment of the Symm3 and the aspect ratio. That is, the Symm3 and the aspect ratio can be adjusted by changing two or more of the conditions of a) to f).

[Catalyst Composition of Catalyst for Producing Unsaturated Aldehyde or Conjugated Diene Compound]

When the catalyst of the present invention is used as a catalyst for producing an unsaturated aldehyde or a conjugated diene compound, the catalytically active component preferably has a composition represented by the following formula (2).

$$Mo_{12}Bi_{b1}Ni_{c1}Co_{d1}Fe_{e1}X_{f1}Y_{g1}Z_{h1}O_{i1} \cdots \qquad (2)$$

(In the formula, Mo, Bi, Ni, Co, and Fe represent molybdenum, bismuth, nickel, cobalt, and iron, respectively, X is at least one element selected from tungsten, antimony, tin, zinc, chromium, manganese, magnesium, calcium, silicon, aluminum, cerium, and titanium, Y is at least one element selected from sodium, potassium, cesium, rubidium, and thallium, Z belongs to Group 1 to Group 16 in the periodic table and means at least one element selected from elements other than above Mo, Bi, Ni, Co, Fe, X, and Y, b1, c1, d1, e1, f1, g1, h1, and i1 represent the numbers of atoms of molybdenum, bismuth, nickel, cobalt, iron, X, Y, Z, and oxygen, respectively, where $0 < b1 \leq 7$, $0 \leq c1 \leq 10$, $0 < d1 \leq 10$, $0 < c1+d1 \leq 20$, $0 < e1 \leq 5$, $0 \leq f1 \leq 2$, $0 \leq g1 \leq 3$, and $0 \leq h1 \leq 5$ are satisfied, and i1 is a value determined by the oxidation state of each element.)

In the above formula (2), preferred ranges of b1 to h1 are as follows.

The lower limit of b1 is 0.1, 0.2, 0.3, 0.4, 0.5, 0.6, or 0.7 in order of preference, and the upper limit thereof is 6.0, 5.0, 4.0, 3.0, 2.0, 1.8, 1.5, 1.2, or 1.0 in order of preference. That is, the range of b1 is preferably 0.1 or more and 6.0 or less, more preferably 0.1 or more and 5.0 or less, more preferably 0.1 or more and 4.0 or less, more preferably 0.2 or more and 3.0 or less, more preferably 0.3 or more and 2.0 or less, more preferably 0.4 or more and 1.8 or less, more preferably 0.5 or more and 1.5 or less, more preferably 0.6 or more and 1.2 or less, and most preferably 0.7 or more and 1.0 or less.

The lower limit of c1 is 0.2, 0.5, 0.8, 1.0, 1.5, or 1.7 in order of preference, and the upper limit thereof is 8.0, 7.0, 6.0, 5.0, 4.0, 3.5, 3.3, 3.0, 2.7, or 2.5 in order of preference. That is, the range of c1 is preferably 0.2 or more and 8.0 or less, more preferably 0.2 or more and 7.0 or less, more preferably 0.2 or more and 6.0 or less, more preferably 0.2 or more and 5.0 or less, more preferably 0.2 or more and 4.0 or less, more preferably 0.5 or more and 3.5 or less, more preferably 0.8 or more and 3.3 or less, more preferably 1.0 or more and 3.0 or less, more preferably 1.5 or more and 2.7 or less, and most preferably 1.7 or more and 2.5 or less.

The lower limit of d1 is 1.0, 2.0, 3.0, 4.0, 5.0, 5.5, or 5.8 in order of preference, and the upper limit thereof is 9.5, 9.0, 8.5, 8.0, 7.5, 7.0, 6.8, or 6.6 in order of preference. That is, the range of d1 is preferably 1.0 or more and 9.5 or less, more preferably 1.0 or more and 9.0 or less, more preferably 2.0 or more and 8.5 or less, more preferably 4.0 or more and 8.0 or less, more preferably 5.0 or more and 7.0 or less, more preferably 5.5 or more and 6.8 or less, and most preferably 5.8 or more and 6.6 or less.

The lower limit of c1+d1 is 1.2, 2.0, 4.0, 6.0, 8.0, or 8.3 in order of preference, and the upper limit thereof is 20.0, 15.0, 12.5, 11.0, 10.0, or 9.0 in order of preference. That is, the range of c1+d1 is preferably 1.2 or more and 20.0 or less, more preferably 2.0 or more and 15.0 or less, more preferably 4.0 or more and 12.5 or less, more preferably 6.0 or more and 11.0 or less, more preferably 8.0 or more and 10.0 or less, and most preferably 8.3 or more and 9.0 or less.

The lower limit of e1 is 0.1, 0.2, 0.5, 0.8, 1.0, 1.5, or 1.6 in order of preference, and the upper limit thereof is 4.5, 4.0, 3.5, 3.0, 2.5, or 2.3 in order of preference. That is, the range of e1 is preferably 0.1 or more and 4.5 or less, more preferably 0.2 or more and 4.5 or less, more preferably 0.5 or more and 3.5 or less, more preferably 0.8 or more and 3.0 or less, more preferably 1.0 or more and 2.5 or less, more preferably 1.5 or more and 2.5 or less, and most preferably 1.6 or more and 2.3 or less.

An upper limit of f1 is 1.8, 1.5, 1.0, 0.8, and 0.5 in order of preference, and a lower limit thereof is preferably 0. That is, a range of f1 is 0 to 1.8, 0 to 1.5, 0 to 1.0, 0 to 0.8, and 0 to 0.5 in order of preference, and f1 is most preferably 0.

The lower limit of g1 is 0.010, 0.020, or 0.030 in order of preference, and the upper limit thereof is 2, 1, 0.5, 0.4, 0.3, 0.2, 0.15, 0.10, 0.075, or 0.050 in order of preference. That is, the range of g1 is preferably 0.010 or more and 2 or less, more preferably 0.010 or more and 1 or less, more preferably 0.010 or more and 0.5 or less, more preferably 0.010 or more and 0.4 or less, more preferably 0.010 or more and 0.3 or less, more preferably 0.010 or more and 0.2 or less, more preferably 0.010 or more and 0.15 or less, more preferably 0.010 or more and 0.10 or less, more preferably 0.020 or more and 0.075 or less, and most preferably 0.030 or more and 0.050 or less.

An upper limit of $h1$ is 4.0, 3.0, 2.0, 1.8, 1.5, 1.0, 0.8, and 0.5 in order of preference, and a lower limit thereof is preferably 0. That is, a range of $h1$ is 0 or more to 4.0 or less, 0 or more to 3.0 or less, 0 or more to 2.0 or less, 0 or more to 1.8 or less, 0 or more to 1.5 or less, 0 or more to 1.0 or less, 0 or more to 0.8 or less, and 0 to 0.5 in order of preference, and $h1$ is most preferably 0.

X in the formula (2) is preferably tungsten, antimony, zinc, magnesium, or cerium, and particularly preferably antimony or zinc.

Y in the formula (2) is preferably sodium, potassium, or cesium, further preferably potassium or cesium, and particularly preferably cesium.

Z in the formula (2) is preferably vanadium, copper, niobium, zirconium, calcium, beryllium, strontium, barium, lead, or phosphorus.

[Method for Producing Catalyst for Producing Unsaturated Aldehyde and Conjugated Diene Compound, etc.]

Starting raw materials for the elements constituting the catalyst represented by the formula (2) above are not particularly limited, but for example, as the raw material for molybdenum component, molybdenum oxides such as molybdenum trioxide, molybdic acid or salts thereof, such as molybdic acid, ammonium paramolybdate, and ammonium metamolybdate, heteropoly acids containing molybdenum or salts thereof, such as phosphomolybdic acid and silicomolybdic acid, and the like can be used.

As the raw material for bismuth component, bismuth salts such as bismuth nitrate, bismuth carbonate, bismuth sulfate, and bismuth acetate, bismuth trioxide, metal bismuth, etc., can be used. These raw materials can be used as solids or as an aqueous solution, a nitric acid solution, or a slurry of bismuth compounds generated from these aqueous solutions. A nitrate, a solution thereof, or a slurry obtained from the solution is preferably used.

As starting raw materials for other component elements, an ammonium salt, a nitrate, a nitrite, a carbonate, a subcarbonate, an acetate, a chloride, an inorganic acid, an inorganic acid salt, a heteropoly acid, a heteropoly acid salt, a sulfate, a hydroxide, an organic acid salt, and an oxide of metallic elements that are typically used in this type of catalyst, or a mixture thereof may be used in combination. An ammonium salt and a nitrate are preferably used.

A compound containing these active components may be used alone or in combination of two or more thereof. A slurry liquid can be obtained by uniformly mixing each active component-containing compound and water. An amount of water to be used in the slurry liquid is not limited as long as it can completely dissolve the entire amount of the compound used or enables uniform mixing. The amount of water to be used may be appropriately determined in consideration of the drying method and drying conditions. The amount of water to be used is usually 100 parts by mass to 2,000 parts by mass with respect to 100 parts by mass of the total mass of the compounds for preparing slurry. The amount of water may be high, but when the amount is too high, the energy cost in the drying step may increase, or drying cannot be completed in some cases.

The slurry liquid of the supply source compounds for the component elements is preferably prepared by (i) a method of mixing the supply source compounds at once, (ii) a method of mixing at once and then performing aging, (iii) a method of mixing in a stepwise manner, (iv) a method of repeating a mixing process and an aging process in a stepwise manner, and a method combining (i) to (iv). Here, the above aging means "an operation in which industrial raw materials or semi-finished products are treated under specific conditions such as a certain period of time and a certain temperature to acquire or improve the required physical properties and chemical properties or proceed with a predetermined reaction". In the present invention, the above certain period of time means a range of 5 minutes or longer and 24 hours or shorter, and the above certain temperature means a range equal to or higher than room temperature and equal to or lower than a boiling point of an aqueous solution or an aqueous dispersion liquid. Among these, in terms of the activity and the yield of the finally obtained catalyst, preferred is (iii) the method of mixing in a stepwise manner, more preferred is a method of preparing a completely dissolved solution for each raw material to be mixed with a mother liquid in a stepwise manner, and most preferred is a method of mixing various mixed solutions of an alkali metal solution and a nitrate with a mother liquid containing a raw material for molybdenum component in a form of a blended solution or slurry. However, it is not always necessary to mix all the catalyst constituent elements in this step, and some elements or some amounts thereof may be added in the subsequent steps.

In the present invention, a shape of stirring blades of a stirrer to be used in mixing essential active component is not limited, and any stirring blades such propeller blades, turbine blades, paddle blades, inclined paddle blades, screw blades, anchor blades, ribbon blades, and large lattice blades can be used in one stage or two or more stages of the same type of blade or different types of blades in an up-down direction. In addition, a baffle (baffle plate) may be installed in a reaction vessel as required.

Next, the slurry liquid thus obtained is dried. The drying method is not limited as long as it is a method capable of completely drying the slurry liquid, and examples thereof include drum drying, freeze drying, spray drying, and evaporation to dryness. Among these, in the present invention, spray drying is particularly preferred because the slurry liquid can be dried into a powder or granules in a short period of time. A drying temperature in the spray drying varies depending on the concentration of the slurry liquid, the liquid feeding rate, or the like, and the temperature at an outlet of a dryer is typically 70°C to 150°C.

The catalyst precursor obtained as described above is preliminary calcined, shaped, and then subjected to main calcination. This allows for controlling and keeping a formed shape of a catalyst to provide a catalyst particularly excellent in mechanical strength for industrial use, and the catalyst achieves stable catalyst performance.

As the shaping, both shaping methods of carrying-shaping of carrying on an inert carrier such as silica and non-carrying-shaping without using a carrier can be used. Specific examples of the shaping method include granulation shaping. As the shape of the shaped product, for example, a spherical shape, an ellipsoid shape, or the like can be appropriately selected in consideration of operating conditions. Preferred is a carried catalyst in which the catalyst precursor is carried on a spherical carrier, particularly an inert carrier such as silica or alumina and which has an average particle diameter of 3.0 mm or more and 10.0 mm or less, and preferably having an average particle diameter of 3.0 mm or more and 8.0 mm or less. That is, a spherical carried catalyst having an average particle diameter of 3.0 mm or more and 8.0 mm or less is most preferable. As for a carrying method, a tumbling granulation, a method using a centrifugal flow coating apparatus, a wash coating and the like, are widely known. The method is not limited as long as the preliminarily calcined powder can be uniformly carried on the carrier. The tumbling granulation is preferred in consideration of the production efficiency of the catalyst, and the like. Specifically, the tumbling granulation is a method of rotating a flat or uneven disc at a high speed in an apparatus that includes the disc at a bottom portion of a fixed cylindrical container, to vigorously stir a carrier charged in the container by repeating the rotation and revolution movements of the carrier, and carrying the powder component on the carrier by adding the preliminarily calcined powder to the container. A binder is preferably used for carrying. Specific examples of the binder which can be used include water, ethanol, methanol, propanol, a polyhydric alcohol, a polyvinyl alcohol that is a polymer-based binder, and an aqueous silica sol solution that is an inorganic binder. Ethanol, methanol, propanol, and a polyhydric alcohol are preferred, a diol such as ethylene glycol and a triol such as glycerin are more preferred, and an aqueous solution having a concentration of glycerin of 5 mass% or more is further preferred. Using an appropriate amount of a glycerin aqueous solution allows for providing a high-performance catalyst having good molding properties and high mechanical strength. An amount of these binders to be used is typically 2 parts by mass to 60 parts by mass with respect to 100 parts by mass of the preliminarily calcined powder, and 15 parts by mass to 50 parts by mass is preferred in the case of using a glycerin aqueous solution. The binder and the preliminarily calcined powder may be alternately supplied to a molding machine or simultaneously supplied to the molding machine during carrying. Further, in the shaping, a small amount of known additives such as graphite and talc may be added. None of a shaping aid, a pore-forming agent, and a carrier added during the shaping is considered as the constituent element of the active component in the present invention, regardless of presence or absence of activity in the sense of converting the raw material into some other product.

The preliminary calcination method, the preliminary calcination conditions, the calcination method, and the calcination conditions are not particularly limited, and known treatment methods and conditions can be applied. The preliminary calcination or the calcination is typically carried out at 200°C or higher and 600°C or lower, and preferably 300°C or higher and 550°C or lower, for 0.5 hours or longer, and preferably 1 hour or longer and 40 hours or shorter under a flow of an oxygen-containing gas such as air or a flow of an inert gas. Here, the inert gas refers to a gas that does not reduce the reaction activity of the catalyst, and specific examples thereof include nitrogen, carbon dioxide, helium, and argon. The optimum conditions for particularly the main calcination vary depending on the reaction conditions at the time of producing an unsaturated aldehyde and/or an unsaturated carboxylic acid using the catalyst or the like, and it is known to those skilled in the art to change the step parameters of the main calcination step, namely, the oxygen content in the atmosphere, the maximum temperature, the calcination time, and the like. Thus, the above change falls within the scope of the present invention. In addition, the calcination step shall be carried out after the above preliminary calcination step, and the maximum temperature (calcination temperature) in the calcination step is higher than the maximum temperature (preliminary calcination temperature) in the above preliminary calcination step. The calcination method is not particularly limited to a fluidized bed, a rotary kiln, a muffle furnace, a tunnel firing furnace, and the like, and an appropriate method should be selected in consideration of the final catalyst performance, the mechanical strength, the shaping properties, the production efficiency, and the like.

The methods for adjusting the SPHT3, the Symm3, and the aspect ratio of the catalyst for producing an unsaturated aldehyde or a conjugated diene compound can also be achieved by appropriately adjusting two or more conditions of preparation, drying, preliminary calcination, pulverization, shaping, and main calcination in the same manner as the catalyst for producing an unsaturated carboxylic acid.

The catalyst of the present invention is preferably used as a catalyst for producing an unsaturated aldehyde compound, an unsaturated carboxylic acid compound, or a conjugated diene compound and is preferably used depending on the composition as described above. Specifically, the catalyst having the composition of the above formula (1) is particularly useful as a catalyst for producing an unsaturated carboxylic acid using an unsaturated aldehyde as a starting raw material. The catalyst having the composition of the above formula (2) is particularly useful as a catalyst for producing an unsaturated aldehyde using propylene, isobutylene, t-butyl alcohol, or the like as a starting raw material or a conjugated diolefin using a monoolefin raw material as a starting raw material. In an exothermic reaction process such as the production of an unsaturated aldehyde compound, an unsaturated carboxylic acid compound, or a conjugated diene compound, different kinds of catalysts may be packed in multiple layers for increasing the activity from the inlet side of a reaction tube to the outlet side of the reaction tube, for the purpose of preventing deterioration of the catalyst itself due to the heat generated by the reaction in an actual plant.

[Use of Catalyst, etc.]

In the method for producing acrolein or acrylic acid using the catalyst of the present invention, the method for circulating the raw material gas may be a normal single flow method or a recycling method, and the method can be carried out under generally used conditions and is not particularly limited. For example, a mixed gas containing 1 to 10 vol% and preferably 4 to 9 vol% of a starting raw material substance at normal temperature, 3 to 20 vol% and preferably 4 to 18 vol% of molecular oxygen, 0 to 60 vol% and preferably 4 to 50 vol% of water vapor, and 20 to 80 vol% and preferably 30 to 60 vol% of an inert gas such as carbon dioxide and nitrogen is introduced, at 240 to 450°C under normal pressure to pressure of 10 atm at a space velocity of 300 to 5000 h$^{-1}$, into the catalyst of the present invention packed in a reaction tube, and the reaction is carried out.

Further, in the method for producing acrolein or acrylic acid, multilayer filling using different types of catalysts is preferable. That is, in the method, a plurality of catalyst layers divided into n and formed in the direction of the raw material gas flow of the reaction tube are provided, and the plurality of types of catalysts are arranged in such a manner that the activity increases from the raw material inlet portion toward the outlet portion in the direction of the raw material gas flow. The number n of the divided layers is not particularly limited but is typically two to five, and preferably two or three. The different types of catalysts not only mean a case where the compositions of the catalysts are different alone but also include a case where the carrying ratios to the inert carrier are different or a case where the dilution rates are different.

EXAMPLES

Hereinafter, the present invention will be described more specifically with Examples, but the present invention is not limited to the Examples unless it deviates from the spirit thereof. In the Examples, the raw material conversion rates, the yields, and the selectivity were calculated according to the following equations.

Raw material conversion rate (%) = (number of moles of reacted acrolein)/(number of moles of supplied acrolein) $\times$ 100

Yield (%) = (number of moles of produced acrylic acid)/(number of moles of supplied acrolein) $\times$ 100

Selectivity (%) = (number of moles of produced acrylic acid)/(number of moles of reacted acrolein) $\times$ 100

The median diameter of each preliminarily calcined powder shown in the Examples is a median value on a volume basis when the equivalent circle diameter $X_{area}$ of the powder dispersed in air was measured using CAMSIZER X2 manufactured by MicrotracBEL Corp. The equivalent circle diameter $X_{area}$ can be calculated by the following equation (IV), where A is the measured particle area.

$$X_{area} = (4A/\pi)^{(1/2)} \cdots (IV)$$

[Example 1]

<Production of Catalyst 1>

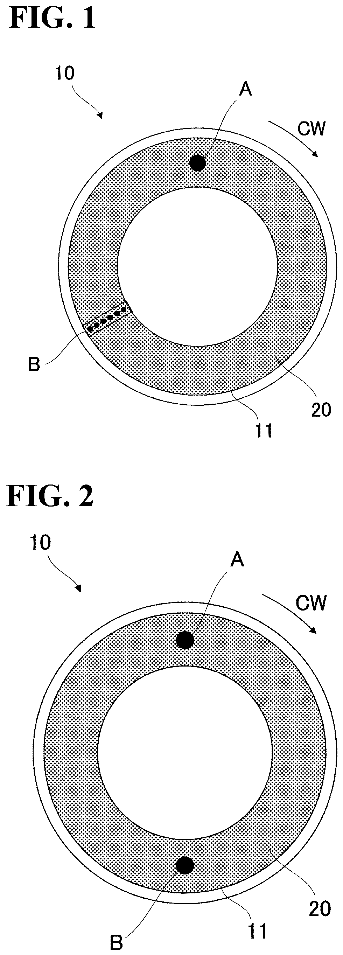

In 5241 parts by mass of pure water heated to 95°C, 148 parts by mass of ammonium paratungstate were completely dissolved. While stirring this solution, 166 parts by mass of ammonium metavanadate, 1000 parts by mass of ammonium molybdate, and 70.5 parts by mass of antimony acetate were then gradually added and thoroughly stirred. Next, a solution obtained by completely dissolving 143 parts by mass of copper sulfate in 427 parts by mass of pure water heated to 80°C was added to the above solution, followed by mixing with stirring. The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a vibration mill to obtain a preliminarily calcined powder (E). The median diameter of the obtained preliminarily calcined powder (E) was 22.7 $\mu$m. To the preliminarily calcined powder (E), 5 mass% of crystalline cellulose and 5 mass% of Milled Fiber EFH150-31 manufactured by Central Glass Co., Ltd. were added and mixed thoroughly, and then the obtained product was carried and molded into a spherical shape on an inert spherical carrier made of a mixture of silica and alumina in such a manner that the carrying ratio became 33 mass% by a tumbling granulation method using a 20 mass% glycerin solution as a binder. A liquid feed pump was used to add the binder. FIG. 1 is a schematic view showing the method for adding the binder in Example 1 and shows the tumbling granulator 10 as viewed from above. The spherical carrier is charged to the tumbling granulator 10, and the carrier is stirred by rotating the bottom plate 11 clockwise as indicated by the arrow CW in FIG. 1. As shown in FIG. 1, granules containing the preliminarily calcined powder (E) were charged at the position A on the bottom plate 11 of the tumbling granulator 10 charged with the spherical carrier. In addition, as shown in FIG. 1, an addition part B of the binder was provided at a position advanced by 240 degrees from the drop point A of the granules in the rotation direction of the bottom plate 11 of the tumbling granulator 10, and the addition port of the binder was divided into six portions in the radius vector direction of the doughnut-shaped catalyst band 20 generated when the bottom plate 11 of the tumbling granulator 10 was rotated to drop the binder from each portion at an even flow rate. The centrifugal acceleration at that time was 4.2 G.

Next, main calcination was performed under the conditions of 375°C and four hours to obtain a spherical catalyst 1 of the present invention.

The composition of the catalyst 1 is $Mo_{12}V_{3.0}W_{1.2}Cu_{1.2}Sb_{0.50}$.

[Example 2]

<Production of Catalyst 2>

In 5241 parts by mass of pure water heated to 95°C, 148 parts by mass of ammonium paratungstate were completely dissolved. While stirring this solution, 166 parts by mass of ammonium metavanadate, 1000 parts by mass of ammonium molybdate, and 70.5 parts by mass of antimony acetate were then gradually added and thoroughly stirred. Next, a solution obtained by completely dissolving 143 parts by mass of copper sulfate in 427 parts by mass of pure water heated to 80°C was added to the above solution, followed by mixing with stirring. The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a ball mill to obtain a preliminarily calcined powder (E). The median diameter of the obtained preliminarily calcined powder (E) was 26.1 $\mu$m. To the preliminarily calcined powder (E), 5 mass% of crystalline cellulose and 5 mass% of Milled Fiber EFH150-31 manufactured by Central Glass Co., Ltd. were added and mixed thoroughly, and then the obtained product was carried and molded into a spherical shape on an inert spherical carrier made of a mixture of silica and alumina in such a manner that the carrying ratio became 33 mass% by a tumbling granulation method using a 20 mass% glycerin solution as a binder. A liquid feed pump having a single flow path was used to add the binder. A single addition port of the binder was provided at a position advanced by 180 degrees from the drop point A of the granules in the rotation direction of the bottom plate 11 of the tumbling granulator 10, and the binder was dropped in the middle in the radius vector direction of the doughnut-shaped catalyst band 20 generated when the bottom plate 11 of the tumbling granulator 10 was rotated. The centrifugal acceleration at that time was 26 G. FIG. 2 is a schematic view showing the method for adding the binder in Example 2 and is the same as FIG. 1 except that the addition position B of the binder is different.

Next, main calcination was performed under the conditions of 390°C and four hours to obtain a spherical catalyst 2 of the present invention.

The composition of the catalyst 2 is $Mo_{12}V_{3.0}W_{1.2}Cu_{1.2}Sb_{0.50}$.

[Example 3]

<Production of Catalyst 3>

In 5241 parts by mass of pure water heated to 95°C, 148 parts by mass of ammonium paratungstate were completely dissolved. While stirring this solution, 166 parts by mass of ammonium metavanadate and 1000 parts by mass of ammonium molybdate were then added, and after observing the dissolution, a suspension obtained by mixing 70.5 parts by mass of antimony acetate and 150 parts by mass of 10 weight% ammonia water was gradually added and thoroughly stirred. Next, a solution obtained by completely dissolving 143 parts by mass of copper sulfate in 427 parts by mass of pure water heated to 80°C was added to the above solution, followed by mixing with stirring. The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a ball mill to obtain a preliminarily calcined powder (E). The median diameter of the obtained preliminarily calcined powder (E) was 26.8 $\mu$m. To the preliminarily calcined powder (E), 5 mass% of crystalline cellulose and 5 mass% of Milled Fiber EFH150-31 manufactured by Central Glass Co., Ltd. were added and mixed thoroughly, and then the obtained product was carried and molded into a spherical shape on an inert spherical carrier made of a mixture of silica and alumina in such a manner that the carrying ratio became 33 mass% by a tumbling granulation method using a 20 mass% glycerin solution as a binder. A liquid feed pump having a single flow path was used to add the binder. A single addition port of the binder was provided at a position advanced by 180 degrees from the drop point A of the granules in the rotation direction of the bottom plate 11 of the tumbling granulator 10, and the binder was dropped in the middle in the radius vector direction of the doughnut-shaped catalyst band 20 generated when the bottom plate 11 of the tumbling granulator 10 was rotated. That is, the addition position of the binder in this example was the same as that in Example 2 shown in FIG. 2. The centrifugal acceleration at that time was 26 G.

Next, main calcination was performed under the conditions of 390°C and four hours to obtain a spherical catalyst 3 of the present invention.

The composition of the catalyst 3 is $Mo_{12}V_{3.0}W_{1.2}Cu_{1.2}Sb_{0.50}$.

[Example 4]

<Production of Catalyst 4>

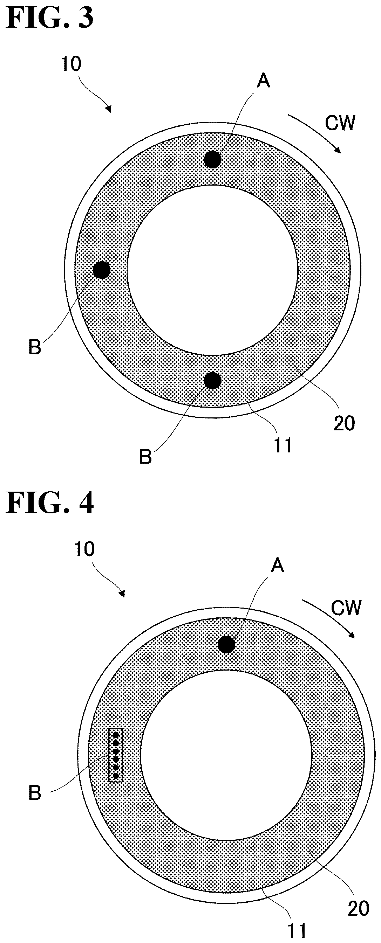

In 5241 parts by mass of pure water heated to 95°C, 148 parts by mass of ammonium paratungstate were completely dissolved. While stirring this solution, 166 parts by mass of ammonium metavanadate and 1000 parts by mass of ammonium molybdate were then added, and after observing the dissolution, 70.5 parts by mass of antimony acetate were gradually added and thoroughly stirred. Next, a solution obtained by completely dissolving 143 parts by mass of copper sulfate in 427 parts by mass of pure water heated to 80°C was added to the above solution, followed by mixing with stirring. The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a ball mill to obtain a preliminarily calcined powder (E). The median diameter of the obtained preliminarily calcined powder (E) was 37.5 $\mu$m. To the preliminarily calcined powder (E), 5 mass% of crystalline cellulose and 5 mass% of Milled Fiber EFH 150-31 manufactured by Central Glass Co., Ltd. were added and mixed thoroughly, and then the obtained product was carried and molded into a spherical shape on an inert spherical carrier made of a mixture of silica and alumina in such a manner that the carrying ratio became 33 mass% by a tumbling granulation method using a 20 mass% glycerin solution as a binder. A liquid feed pump having two flow paths was used to add the binder. Single addition ports of the binder were provided at positions advanced by 180 degrees and 270 degrees from the drop point A of the granules in the rotation direction of the bottom plate 11 of the tumbling granulator 10, and the binder was dropped in the middle in the radius vector direction of the doughnut-shaped catalyst band 20 generated when the bottom plate 11 was rotated. The centrifugal acceleration at that time was 10 G. FIG. 3 is a schematic view showing the method for adding the binder in Example 4 and is the same as FIG. 1 except that the addition positions B of the binder are different.

Next, main calcination was performed under the conditions of 390°C and four hours to obtain a spherical catalyst 4 of the present invention.

The composition of the catalyst 4 is $Mo_{12}V_{3.0}W_{1.2}Cu_{1.2}Sb_{0.50}$.

[Example 5]

<Production of Catalyst 5>

In 5241 parts by mass of pure water heated to 95°C, 148 parts by mass of ammonium paratungstate were completely dissolved. While stirring this solution, 166 parts by mass of ammonium metavanadate and 1000 parts by mass of ammonium molybdate were then added, and after observing the dissolution, 70.5 parts by mass of antimony acetate were gradually added and thoroughly stirred. Next, a solution obtained by completely dissolving 143 parts by mass of copper sulfate in 427 parts by mass of pure water heated to 80°C was added to the above solution, followed by mixing with stirring. The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a ball mill to obtain a preliminarily calcined powder (E). The median diameter of the obtained preliminarily calcined powder (E) was 37.5 $\mu$m. To the preliminarily calcined powder (E), 5 mass% of crystalline cellulose and 5 mass% of Milled Fiber EFH 150-31 manufactured by Central Glass Co., Ltd. were added and mixed thoroughly, and then the obtained product was carried and molded into a spherical shape on an inert spherical carrier made of a mixture of silica and alumina in such a manner that the carrying ratio became 33 mass% by a tumbling granulation method using a 20 mass% glycerin solution as a binder. A liquid feed pump having a single flow path was used to add the binder. A single addition port of the binder was provided at a position advanced by 180 degrees from the drop point A of the granules in the rotation direction of the bottom plate 11 of the tumbling granulator 10, and the binder was dropped in the middle in the radius vector direction of the doughnut-shaped catalyst band 20 generated when the bottom plate 11 of the tumbling granulator 10 was rotated. That is, the addition position of the binder in this example was the same as that in Example 2 shown in FIG. 2. The centrifugal acceleration at that time was 26 G.

Next, main calcination was performed under the conditions of 390°C and four hours to obtain a spherical catalyst 5 of the present invention.

The composition of the catalyst 5 is $Mo_{12}V_{3.0}W_{1.2}Cu_{1.2}Sb_{0.50}$.

[Example 6]

<Production of Catalyst 6>

In 5241 parts by mass of pure water heated to 95°C, 148 parts by mass of ammonium paratungstate were completely dissolved. While stirring this solution, 166 parts by mass of ammonium metavanadate and 1000 parts by mass of ammonium molybdate were then added, and after observing the dissolution, 70.5 parts by mass of antimony acetate were gradually added and thoroughly stirred. Next, a solution obtained by completely dissolving 143 parts by mass of copper sulfate in 427 parts by mass of pure water heated to 80°C was added to the above solution, followed by mixing with stirring. The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a ball mill to obtain a preliminarily calcined powder (E). The median diameter of the obtained preliminarily calcined powder (E) was 36.0 μm. To the preliminarily calcined powder (E), 5 mass% of crystalline cellulose was added and mixed thoroughly, and then the obtained product was carried and molded into a spherical shape on an inert spherical carrier made of a mixture of silica and alumina in such a manner that the carrying ratio became 33 mass% by a tumbling granulation method using a 20 mass% glycerin solution as a binder. A liquid feed pump was used to add the binder. The center position of the addition port of the binder was set at a position advanced by 270 degrees from the drop point A of the granules in the rotation direction of the bottom plate 11 of the tumbling granulator 10, and the addition port of the binder was divided into six portions in the direction perpendicular to the radius vector direction of the doughnut-shaped catalyst band 20 generated when the bottom plate 11 of the tumbling granulator 10 was rotated to drop the binder from each portion at an even flow rate. The centrifugal acceleration at that time was 5.5 G. FIG. 4 is a schematic view showing the method for adding the binder in Example 6 and is the same as FIG. 1 except that the addition positions B of the binder are different.

Next, main calcination was performed under the conditions of 390°C and four hours to obtain a spherical catalyst 6 of the present invention.

The composition of the catalyst 6 is $Mo_{12}V_{3.0}W_{1.2}Cu_{1.2}Sb_{0.50}$.

[Comparative Example 1]

<Production of Catalyst 7>

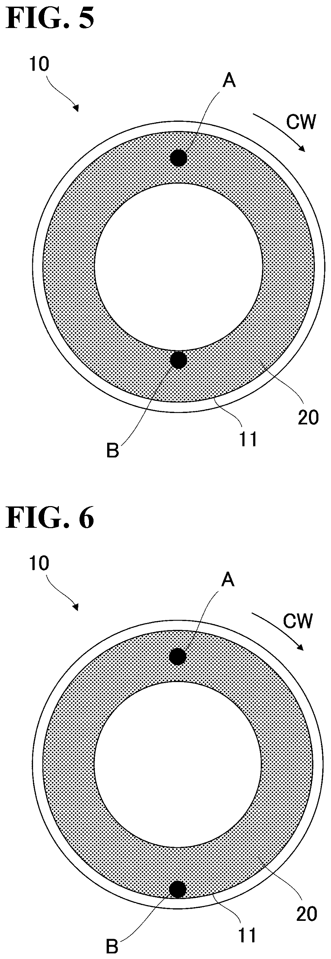

In 5241 parts by mass of pure water heated to 95°C, 148 parts by mass of ammonium paratungstate were completely dissolved. While stirring this solution, 166 parts by mass of ammonium metavanadate and 1000 parts by mass of ammonium molybdate were then added, and after observing the dissolution, 70.5 parts by mass of antimony acetate were gradually added and thoroughly stirred. Next, a solution obtained by completely dissolving 143 parts by mass of copper sulfate in 427 parts by mass of pure water heated to 80°C was added to the above solution, followed by mixing with stirring. The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a ball mill to obtain a preliminarily calcined powder (E). The median diameter of the obtained preliminarily calcined powder (E) was 29.8 μm. To the preliminarily calcined powder (E), 5 mass% of crystalline cellulose and 5 mass% of Milled Fiber EFH150-31 manufactured by Central Glass Co., Ltd. were added and mixed thoroughly, and then the obtained product was carried and molded into a spherical shape on an inert spherical carrier made of a mixture of silica and alumina in such a manner that the carrying ratio became 33 mass% by a tumbling granulation method using a 20 mass% glycerin solution as a binder. A liquid feed pump having a single flow path was used to add the binder. A single addition port of the binder was provided at a position advanced by 180 degrees from the drop point A of the granules in the rotation direction of the bottom plate 11 of the tumbling granulator 10, and the binder was dropped at a position close to the center of the rotation (the internal circumference of the catalyst band 20 in the radius vector direction) on the doughnut-shaped catalyst band 20 generated when the bottom plate 11 of the tumbling granulator 10 was rotated. The centrifugal acceleration at that time was 26 G. FIG. 5 is a schematic view showing the method for adding the binder in Comparative Example 1 and is the same as FIG. 1 except that the addition position B of the binder is different.

Next, main calcination was performed under the conditions of 390°C and four hours to obtain a spherical catalyst 7.

The composition of the catalyst 7 is $Mo_{12}V_{3.0}W_{1.2}Cu_{1.2}Sb_{0.50}$.

[Comparative Example 2]

<Production of Catalyst 8>

In 5241 parts by mass of pure water heated to 95°C, 148 parts by mass of ammonium paratungstate were completely dissolved. While stirring this solution, 166 parts by mass of ammonium metavanadate and 1000 parts by mass of ammonium molybdate were then added, and after observing the dissolution, 70.5 parts by mass of antimony acetate were gradually added and thoroughly stirred. Next, a solution obtained by completely dissolving 143 parts by mass of copper sulfate in 427 parts by mass of pure water heated to 80°C was added to the above solution, followed by mixing with stirring. The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a ball mill to obtain a preliminarily calcined powder (E). The median diameter of the obtained preliminarily calcined powder (E) was 28.2 $\mu$m. To the preliminarily calcined powder (E), 5 mass% of crystalline cellulose and 5 mass% of Milled Fiber EFH 150-31 manufactured by Central Glass Co., Ltd. were added and mixed thoroughly, and then the obtained product was carried and molded into a spherical shape on an inert spherical carrier made of a mixture of silica and alumina in such a manner that the carrying ratio became 25 mass% by a tumbling granulation method using a 20 mass% glycerin solution as a binder. A liquid feed pump having a single flow path was used to add the binder. A single addition port of the binder was provided at a position advanced by 180 degrees from the drop point A of the granules in the rotation direction of the bottom plate 11 of the tumbling granulator 10, and the binder was dropped on the wall side of the tumbling granulator 10 (the outer circumference of the catalyst band 20 in the radius vector direction) on the doughnut-shaped catalyst band 20 generated when the bottom plate 11 of the tumbling granulator 10 was rotated. The centrifugal acceleration at that time was 26 G. FIG. 6 is a schematic view showing the method for adding the binder in Comparative Example 2 and is the same as FIG. 1 except that the addition position B of the binder is different.

Next, main calcination was performed under the conditions of 390°C and four hours to obtain a spherical catalyst 8.

The composition of the catalyst 8 is $Mo_{12}V_{3.0}W_{1.2}Cu_{1.2}Sb_{0.50}$.

[Comparative Example 3]

<Production of Catalyst 9>

In 5241 parts by mass of pure water heated to 95°C, 148 parts by mass of ammonium paratungstate were completely dissolved. While stirring this solution, 166 parts by mass of ammonium metavanadate and 1000 parts by mass of ammonium molybdate were then added, and after observing the dissolution, 70.5 parts by mass of antimony acetate were gradually added and thoroughly stirred. Next, a solution obtained by completely dissolving 137 parts by mass of copper nitrate in 410 parts by mass of pure water heated to 80°C was added to the above solution, followed by mixing with stirring. The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a ball mill to obtain a preliminarily calcined powder (E). The median diameter of the obtained preliminarily calcined powder (E) was 17.0 $\mu$m. To the preliminarily calcined powder (E), 5 mass% of crystalline cellulose and 5 mass% of Milled Fiber EFH 150-31 manufactured by Central Glass Co., Ltd. were added and mixed thoroughly, and then the obtained product was carried and molded into a spherical shape on an inert spherical carrier made of a mixture of silica and alumina in such a manner that the carrying ratio became 33 mass% by a tumbling granulation method using a 20 mass% glycerin solution as a binder. A liquid feed pump having a single flow path was used to add the binder. A single addition port of the binder was provided at a position advanced by 180 degrees from the drop point A of the granules in the rotation direction of the bottom plate 11 of the tumbling granulator 10, and the binder was dropped in the middle in the radius vector direction of the doughnut-shaped catalyst band 20 generated when the bottom plate 11 of the tumbling granulator 10 was rotated. That is, the addition position of the binder in this example was the same as that in Example 2 shown in FIG. 2. The centrifugal acceleration at that time was 26 G.

Next, main calcination was performed under the conditions of 390°C and four hours to obtain a spherical catalyst 9.

The composition of the catalyst 9 is $Mo_{12}V_{3.0}W_{1.2}Cu_{1.2}Sb_{0.50}$.

Oxidation reaction was carried out using the obtained catalysts 1 to 9. A reaction tube having an inner diameter of 28.4 mm was filled with 67.6 ml of the catalyst, and a gas having the following composition obtained by catalytic gas-phase oxidation of propylene using a molybdenum-bismuth-based catalyst was introduced. A reaction was performed at SV (space velocity; flow rate of raw material gas per unit time/apparent volume of packed catalyst) of 1020/hr and a reaction bath temperature of 260°C.

| | |
|---|---|
| Acrolein | 5.9 vol% |
| Unreacted propylene + other organic compound | 1.7 vol% |
| Oxygen | 4.7 vol% |
| Steam | 17.2 vol% |
| Nitrogen-co ntaining inert gas | 70.5 vol% |

The reaction results obtained by the oxidation reaction and the particle shape parameters of the catalysts obtained by dynamic image analysis using CAMSIZER X2 manufactured by MicrotracBEL Corp. are shown in Table 1.

[Table 1]

| Catalyst Name | Raw Material Conversion Rate (%) | Selectivity (%) | SPHT3 | Symm3 | b/l3 |
|---|---|---|---|---|---|
| Catalyst 1 | 99.4 | 96.7 | 0.9758 | 0.9749 | 0.9271 |
| Catalyst 2 | 99.0 | 97.1 | 0.9757 | 0.9757 | 0.9379 |
| Catalyst 3 | 98.8 | 96.9 | 0.9800 | 0.9792 | 0.9454 |
| Catalyst 4 | 98.8 | 97.2 | 0.9807 | 0.9783 | 0.9408 |
| Catalyst 5 | 98.5 | 97.2 | 0.9807 | 0.9799 | 0.9388 |
| Catalyst 6 | 98.1 | 97.4 | 0.9316 | 0.9713 | 0.9361 |
| Catalyst 7 | 96.6 | 97.1 | 0.9821 | 0.9862 | 0.9543 |
| Catalyst 8 | 95.1 | 97.9 | 0.9826 | 0.9854 | 0.9571 |
| Catalyst 9 | 78.9 | 98.0 | 0.9857 | 0.9894 | 0.9631 |

[Example 7] (Preparation of Catalyst 10)

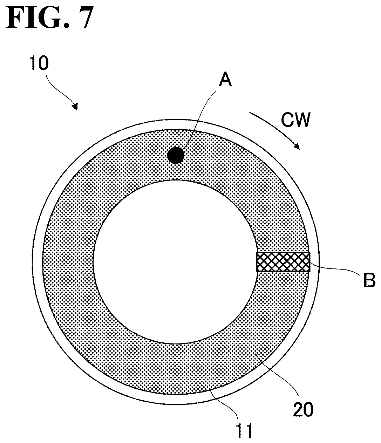

In 380 parts by mass of pure water heated to 60°C, 100 parts by mass of ammonium heptamolybdate was completely dissolved (mother liquid 1). Next, 0.37 parts by mass of cesium nitrate was dissolved in 3.3 parts by mass of pure water, and the solution was added to the mother liquid 1. Next, 37 parts by mass of ferric nitrate, 90 parts by mass of cobalt nitrate, and 33 parts by mass of nickel nitrate were dissolved in 85 parts by mass of pure water heated to 80°C, and the solution was added to the mother liquid 1. Subsequently, 21 parts by mass of bismuth nitrate were dissolved in a nitric acid aqueous solution prepared by adding 5.4 parts by mass of nitric acid (60 mass%) to 23 parts by mass of pure water heated to 80°C, and the solution was added to the mother liquid 1. This mother liquid 1 was dried by a spray drying method, and the obtained dry powder was subjected to preliminary calcination under conditions of 440°C and 4 hours. The median diameter of thus obtained preliminarily calcined powder was 36.3 μm, and the next step was conducted without pulverization. To the preliminarily calcined powder, 5 mass% of crystalline cellulose was added and mixed thoroughly, and then the obtained product was carried into a spherical shape on an inert carrier made of a mixture of silica and alumina in such a manner that the carrying ratio became 50 mass% by a tumbling granulation method using a 33 mass% glycerin solution as a binder. A sprayer was used to add the binder, and the binder was sprayed in such a manner that the binder was evenly applied to the catalyst passing the position advanced by 90 degrees from the drop point A of the granules in the rotation direction of the bottom plate 11 of the tumbling granulator 10. The centrifugal acceleration at that time was 26 G. FIG. 7 is a schematic view showing the method for adding the binder in Example 7 and is the same as FIG. 1 except that the addition position B of the binder is different.

Next, main calcination was performed under the conditions of 520°C and four hours to obtain a catalyst 10. The composition of the catalyst 10 was Mo:Bi:Fe:Co:Ni:Cs = 12:0.93:2.0:6.5:2.4:0.040.

Oxidation reaction was carried out using the obtained catalyst 10. A reaction tube having an inner diameter of 28.4 mm was filled with 50.7 ml of the catalyst. A gas having the following composition was caused to flow at SV (space velocity; flow rate of raw material gas per unit time/apparent volume of packed catalyst) of 1190/hr, and a reaction was performed at a reaction bath temperature of 315°C.

| | |
|---|---|
| Propylene | 8.0 vol% |
| Oxygen | 13.6 vol% |
| Steam | 8.0 vol% |
| Nitrogen-containing inert gas | 70.4 vol% |

The reaction results obtained by the oxidation reaction and the particle shape parameters of the catalyst obtained by dynamic image analysis using CAMSIZER X2 manufactured by MicrotracBEL Corp. are shown in Table 2. The reaction results are values calculated by the following equations.

Propylene conversion rate (%) = (number of moles of reacted propylene)/(number of moles of supplied propylene) $\times$ 100

Useful yield (%) = (total number of moles of acrolein and acrylic acid produced)/(number of moles of propylene supplied) $\times$ 100

[Table 2]

| Catalyst Name | Propylene Conversion Rate (%) | Useful Yield (%) | SPHT3 | Symm3 | b/l3 |
|---|---|---|---|---|---|
| Catalyst 10 | 65.3 | 62.7 | 0.9755 | 0.9821 | 0.9507 |

The present application is based on Japanese Patent Application No. 2022-202842 filed on December 20, 2022, contents of which are incorporated herein by reference.

INDUSTRIAL APPLICABILITY

According to the present invention, the catalytic activity can be maintained high, in particular when producing acrylic acid by subjecting acrolein as a raw material to a catalytic gas-phase oxidation reaction. Therefore, this allows a plant for producing acrylic acid to be stably operated for a long period of time, which is very useful.

**Claims**

1. A catalyst having a value of a sphericity parameter SPHT3 obtained by particle shape measurement by a dynamic image analysis method of 0.9820 or less,

    wherein the sphericity parameter SPHT3 is a value obtained by the following equation (I):

    $$SPHT3 = 4\pi A/P^2 \cdots(I),$$

    and
    wherein in the equation (I), A is an area of a particle photographed by the dynamic image analysis method, and P is a perimeter of the particle.

2. The catalyst according to claim 1, comprising a catalytically active component represented by the following formula (1):

    $$(Mo)_{12}(V)_a(W)_b(Cu)_c(Sb)_d(X)_e(Y)_f(Z)_g(O)_h \qquad (1),$$

    where, in the formula, Mo, V, W, Cu, Sb, and O represent molybdenum, vanadium, tungsten, copper, antimony, and oxygen, respectively; X represents at least one element selected from the group consisting of an alkali metal and thallium; Y represents at least one element selected from the group consisting of magnesium, calcium, strontium, barium, and zinc; Z represents at least one element selected from the group consisting of bismuth, tellurium, silver, selenium, silicon, aluminum, boron, niobium, cerium, tin, chromium, manganese, iron, cobalt, nickel, samarium, germanium, zirconium, titanium, tantalum, lead, indium, sulfur, palladium, gallium, lanthanum, and arsenic; a, b, c, d, e, f, g, and h represent the atomic ratios of the respective elements; a satisfies $0 < a \leq 10.0$, b satisfies $0 \leq b \leq 10.0$, c satisfies $0 < c \leq 6.0$, d satisfies $0 \leq d \leq 10.0$, e satisfies $0 \leq e \leq 0.50$, f satisfies $0 \leq f \leq 1.0$, and g satisfies $0 \leq g < 6.0$, with respect to molybdenum atom being 12; and h is the number of oxygen atoms necessary for satisfying the valence of each component.

3. The catalyst according to claim 2, wherein in the formula (1), $1.0 \leq a \leq 5.0$, $0.50 \leq b \leq 3.0$, $0.50 \leq c \leq 3.0$, and $0 < d \leq 2.0$ are satisfied.

4. The catalyst according to claim 1, comprising a catalytically active component represented by the following formula (2):

$$Mo_{12}Bi_{b1}Ni_{c1}Co_{d1}Fe_{e1}X_{f1}Y_{g1}Z_{h1}O_{i1} \cdots \qquad (2),$$

where, in the formula, Mo, Bi, Ni, Co, and Fe represent molybdenum, bismuth, nickel, cobalt, and iron, respectively; X is at least one element selected from tungsten, antimony, tin, zinc, chromium, manganese, magnesium, calcium, silicon, aluminum, cerium, and titanium; Y is at least one element selected from sodium, potassium, cesium, rubidium, and thallium; Z belongs to Group 1 to Group 16 in the periodic table and means at least one element selected from elements other than above Mo, Bi, Ni, Co, Fe, X, and Y; b1, c1, d1, e1, f1, g1, h1, and i1 represent the numbers of atoms of molybdenum, bismuth, nickel, cobalt, iron, X, Y, Z, and oxygen, respectively; $0 < b1 \leq 70$, $0 \leq c1 \leq 10.0$, $0 < d1 \leq 10.0$, $0 < c1+d1 \leq 20.0$, $0 < e1 \leq 5.0$, $0 \leq f1 \leq 2.0$, $0 \leq g1 \leq 3.0$, and $0 \leq h1 \leq 5.0$ are satisfied; and i1 is a value determined by the oxidation state of each element.

5. The catalyst according to claim 4, wherein in the formula (2), $0.50 \leq b1 \leq 1.7$, $1.0 \leq c1 \leq 3.3$, $5.0 \leq d1 \leq 6.8$, and $1.0 \leq e1 \leq 3.0$ are satisfied.

6. The catalyst according to any one of claims 1 to 5, wherein a catalytically active component is carried on an inert carrier.

7. The catalyst according to claim 6, wherein the inert carrier is silica, alumina, or a combination thereof.

8. The catalyst according to any one of claims 1 to 5, wherein the catalyst is used for producing an unsaturated aldehyde, an unsaturated carboxylic acid, or a conjugated diene compound.

9. A method for producing an unsaturated aldehyde, an unsaturated carboxylic acid, or a conjugated diene compound using the catalyst according to any one of claims 1 to 5.

# FIG. 1

# FIG. 2

# FIG. 3

# FIG. 4

# FIG. 5

# FIG. 6

# FIG. 7

## INTERNATIONAL SEARCH REPORT

International application No.

**PCT/JP2023/044644**

### A. CLASSIFICATION OF SUBJECT MATTER

*B01J 35/51*(2024.01)i; *B01J 23/887*(2006.01)i; *B01J 23/888*(2006.01)i; *C07B 61/00*(2006.01)i; *C07C 45/35*(2006.01)i; *C07C 47/22*(2006.01)i; *C07C 51/25*(2006.01)i; *C07C 57/05*(2006.01)i

FI:  B01J35/51; B01J23/887 Z; B01J23/888 Z; C07B61/00 300; C07C45/35; C07C47/22 C; C07C51/25; C07C57/05

According to International Patent Classification (IPC) or to both national classification and IPC

### B. FIELDS SEARCHED

Minimum documentation searched (classification system followed by classification symbols)

B01J35/51; B01J23/887; B01J23/888; C07B61/00; C07C45/35; C07C47/22; C07C51/25; C07C57/05

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JST7580/JSTChina (JDreamIII)

### C. DOCUMENTS CONSIDERED TO BE RELEVANT

| Category* | Citation of document, with indication, where appropriate, of the relevant passages | Relevant to claim No. |
|---|---|---|
| X | JP 2009-131776 A (NIPPON SHOKUBAI CO., LTD.) 18 June 2009 (2009-06-18) claims, paragraphs [0016]-[0018], [0027] | 1-3, 6-9 |
| X | WO 2014/073419 A1 (MITSUBISHI RAYON CO., LTD.) 15 May 2014 (2014-05-15) claims, paragraphs [0019]-[0020], [0054]-[0059] | 1, 4-9 |
| X | EP 4052788 A1 (CHINA PETROLEUM & CHEMICAL CORPORATION) 07 September 2022 (2022-09-07) claims, paragraphs [0186]-[0222], table 1 | 1, 6-7 |
| A | JP 2009-285581 A (ASAHI KASEI CHEMICALS CORPORATION) 10 December 2009 (2009-12-10) entire text | 1-9 |

☐ Further documents are listed in the continuation of Box C.  ☑ See patent family annex.

| | |
|---|---|
| *     Special categories of cited documents:<br>"A"  document defining the general state of the art which is not considered to be of particular relevance<br>"D"  document cited by the applicant in the international application<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **02 February 2024** | **27 February 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

EP 4 640 312 A1

## INTERNATIONAL SEARCH REPORT
### Information on patent family members

International application No.

**PCT/JP2023/044644**

| Patent document cited in search report | | | Publication date (day/month/year) | Patent family member(s) | Publication date (day/month/year) |
|---|---|---|---|---|---|
| JP | 2009-131776 | A | 18 June 2009 | (Family: none) | |
| WO | 2014/073419 | A1 | 15 May 2014 | US 2015/0274626 A1<br>claims, paragraphs [0021]-[0022], [0057]-[0064]<br>CN 104781221 A<br>KR 10-2015-0081259 A | |
| EP | 4052788 | A1 | 07 September 2022 | US 2022/0387984 A1<br>claims, paragraphs [0250]-[0306], table 1<br>CN 112742401 A | |
| JP | 2009-285581 | A | 10 December 2009 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H08299797 A
- JP 2003251184 A
- JP 2015120133 A
- JP 2001079408 A

- WO 2012073584 A
- JP 2009214105 A
- JP 2015096497 A
- JP 2022202842 A